# EUROPEAN PATENT APPLICATION

(11) **EP 2 463 383 A2**
(43) Date of publication of application: **13.06.2012**
(21) Application number: 11189045.5
(22) Date of filing: 06.11.2008
(51) Int. Cl.: C12Q 1/68

(54) **Gene expression signatures for chronic/sclerosing allograft nephropathy**

(30) Priority: 08.11.2007 EP 07120263
(62) Divisional of application: 08846586.9
(71) Applicant: Novartis AG, 4056 Basel (CH)
(72) Inventor: Saint-Mezard, Pierre, F-68220 Hesingue (FR); Zhang, Hai, CH-4103 Bottmingen (CH)
(74) Representative: Graff, Alan

(57) **Abstract**

The invention relates to the analysis and identification of genes that are regulated simultaneously in chronic kidney transplant rejection. This simultaneous regulation of genes provides a molecular signature to accurately detect, and optionally classify, chronic kidney transplant rejection.

## Description

### FIELD OF THE INVENTION

This invention relates generally to the analytical testing of tissue samples *in vitro*, and more particularly to gene- or protein-based tests useful in clinical diagnosis of chronic allograft nephropathy.

### BACKGROUND OF THE INVENTION

The success of kidney transplantation leads to one-year graft survival rates in the order of 90% and correlates with the development of powerful immunosuppressive agents to prevent and to treat acute transplant rejection ("AR") episodes. Chronic transplant dysfunction is a phenomenon in solid organ transplants displaying a gradual deterioration of graft function months to years after transplantation, eventually leading to graft failure, and which is accompanied by characteristic histological features. Clinically, chronic transplant dysfunction in kidney grafts, *e.g.*, chronic/sclerosing allograft nephropathy ("CAN") is the the leading causes of late graft loss (around 3-5% per year). CAN (also known as chronic rejection (CR), manifests itself as a slowly progressive decline in glomerular filtration rate, usually in conjunction with proteinuria and arterial hypertension. This disorder represents a consequence of combined immunological injury (*e.g*., chronic rejection) and non-immunological damage (*e.g.*, hypertensive nephrosclerosis, or nephrotoxicity of immuno-suppressants like cyclosporine A), ultimately leading to fibrosis and sclerosis of the allograft, associated with progressive loss of kidney function. Despite clinical application of potent immunoregulatory drugs and biologic agents, chronic rejection remains a common and serious post-transplantation complication. Chronic rejection is a relentlessly progressive process. There exists no satisfactory therapy yet for CAN.

The single most common cause for early graft failure, especially within one month post-transplantation, is immunologic rejection of the allograft. The unfavorable impact of the rejection is magnified by the fact that: (a) the use of high-dose anti-rejection therapy, superimposed upon maintenance immunosuppression, is primarily responsible for the morbidity and mortality associated with transplantation, (b) the immunization against "public" HLA-specificities resulting from a rejected graft renders this patient population difficult to retransplant and (c) the return of the immunized recipient with a failed graft to the pool of patients awaiting transplantation enhances the perennial problem of organ shortage.

Histopathological evaluation of biopsy tissue is the gold standard for the diagnosis of CAN, while prediction of the onset of CAN is currently impossible. CAN diagnosis is often based on observer-dependent interpretation of unspecific histological alterations, and patient prognosis remains ill-defined.

The differentiation of the diagnosis of rejection, *e.g.*, CAN, from other etiologies for graft dysfunction and institution of effective therapy is a complex process because: (a) the percutaneous core needle biopsy of grafts, the best of available current tools to diagnose rejection is performed usually after the "fact", *i.e.*, graft dysfunction and graft damage (irreversible in some instances) are already present, (b) the morphological analysis of the graft provides modest clues with respect to the potential for reversal of a given rejection episode, and minimal clues regarding the likelihood of recurrence ("rebound"), and (c) the mechanistic basis of the rejection phenomenon, a prerequisite for the design of therapeutic strategies, is poorly defined by current diagnostic indices, including morphologic features of rejection.

The diagnosis of, for example, renal allograft rejection is made usually by the development of graft dysfunction (*e.g.*, an increase in the concentration of serum creatinine) and morphologic evidence of graft injury in areas of the graft also manifesting mononuclear cell infiltration. Two caveats apply, however, to the use of abnormal renal function as an indicator of the rejection process: first, deterioration in renal function is not always available as a clinical clue to diagnose rejection since many of the cadaveric renal grafts suffer from acute (reversible) renal failure in the immediate post-transplantation period due to injury from harvesting and *ex vivo* preservation procedures. Second, even when immediately unimpaired renal function is present, graft dysfunction might develop due to a non-immunologic cause, such as immunosuppressive therapy itself.

For example, cyclosporine (CsA) nephrotoxicity, a complication that is not readily identified solely on the basis of plasma/blood concentrations of CsA, is a common complication. The clinical importance of distinguishing rejection from CsA nephrotoxicity cannot be overemphasized since the therapeutic strategies are diametrically opposite: escalation of immunosuppressants for rejection, and reduction of CsA dosage for nephrotoxicity.

As such, the current monitoring and diagnostic modalities are ill-suited to the diagnosis of CAN, particularly at an early stage and new strategies are needed to improve long-term graft survival. Accordingly, a need exists for identifying gene- or protein-based tests that are more sensitive and which can be used in clinical diagnosis of rejection, especially in its early and/or pre-clinical state. Specifically, molecular diagnostics, like gene expression profiling, may aid to further refine the BANFF 97 disease classification (Racusen, et al., 1999, Kidney Int. 55(2):713-23), and may also be employed as predictive or early diagnostic biomarkers. Accurately predicting and diagnosing the outcome of CAN episode is crucial in the trend to optimize treatments and prevent the development of CAN and kidney loss of function.

### Summary

The present invention is based, in part, on the finding that increased or decreased expression of one or more genes and/or the encoded proteins can be reliably associated with certain graft rejection states. Thus, as a result of the data described herein, methods are now available for the rapid and reliable diagnosis of acute and chronic rejection, even in cases where allograft biopsies show only mild cellular infiltrates.

Described herein for the first time is an analysis of genes that are up-regulated and/or down-regulated simultaneously, and which provide a "molecular signature" to accurately detect and/or grade transplant chronic rejection. Early diagnosis of allograft rejection (*e.g.*, renal allograft rejection) and new prognostic markers are important to minimize and personalize immunosuppression. In addition to histopathological differential diagnosis, gene expression profiling significantly improves disease classification by defining these molecular signatures.

Thus, the study results presented herein demonstrate the ability to use molecular signature analysis to differentiate classes of allograft rejection. For the first time, this work has showed that a molecular signature could differentiate biopsies according to gradual severity of CAN (I, II, III). The list of 33 probesets/genes identified in this analysis, correctly classified different CAN grades with 90% accuracy in human and 100% accuracy in a NHP study of CAV. It is difficult to achieve a lower misclassification rate because of the inherent ambiguity of the visual histopathological diagnosis. Differences between CAN grade I and II are slight and prone to subjective weighting by individual pathologists. This trend is well reflected by gene expression. For example, while grade III samples are easily separated, several samples could be classified as either grade I or II. In this respect, grade I and grade II samples are often differentially classified by the PAM and SVM algorithms (data not shown). Early differential diagnosis will be important for intervention to lessen CAN before the putatively irreversible grade III manifests, taken into account that 25% of patients demonstrate grade II CAN one-year post-transplantation).

Accordingly, methods and compositions for monitoring the status of a transplanted organ in a subject are described herein. This involves evaluating transplant rejection in a subject by determining the level (*i.e.*, magnitude) of gene expression in a post-transplant sample obtained from the subject and comparing the relative expression of the marker genes to a baseline level of the marker. Regulation of gene expression (*i.e.*, increased or decreased gene expression) of a plurality of selected genes in the sample indicates rejection.

Altered expression of the combination of the gene markers identified, which are listed in Table 1, indicates transplant rejection. In one embodiment, increased expression of one, two or more genes of any of the genes of Table 1, preferably at least 5, 10, 15 or 18 of the genes of Table 1, indicates transplant rejection (and in some embodiments also enables the grade of CAN to be determined or estimated). In another embodiment, decreased expression of one, two or more genes of any of the genes of Table 1, preferably at least 5, 10, or 15 of the genes of Table 1, indicates transplant rejection (and in some embodiments also enables the grade of CAN to be determined or estimated). Typically, a range of markers are selected, some of which show decreased expression and some of which show increased expression compared to control values.

Accordingly, in one aspect, the present invention provides a method for assessing the onset of a chronic rejection of a transplanted kidney in a subject, comprising the steps of:
(a) obtaining a post-transplantation sample from the subject;
(b) determining the level of expression in the post-transplantation sample of a combination of a plurality of genes selected from the group consisting of the genes identified in Table 1;
(c) comparing the level of gene expression of said plurality of genes in the post-transplantation sample with the magnitude of gene expression of the same genes in a control sample to generate a differential expression profile; and
(d) comparing the differential expression profile with one or more reference differential expression profiles indicative of one or more stages of chronic/sclerosing allograft nephropathy,
thereby assessing the onset of rejection of the transplanted organ in the subject.

In a related aspect, the present invention provides a method of classifying the stage of chronic/sclerosing allograft nephropathy in a subject suffering from chronic rejection of a transplanted kidney, comprising the steps of:
(a) obtaining a post-transplantation sample from the subject;
(b) determining the level of expression in the post-transplantation sample of a combination of a plurality of genes selected from the group consisting of the genes identified in Table 1;
(c) comparing the level of gene expression of said plurality of genes in the post-transplantation sample with the magnitude of gene expression of the same genes in a control sample to generate a differential expression profile; and
(d) comparing the differential expression profile with one or more reference differential expression profiles indicative of one or more stages of chronic/sclerosing allograft nephropathy,
thereby classifying the stage of chronic/sclerosing allograft nephropathy in the subject.

In a preferred embodiment, the plurality of genes selected have a expression profile that distinguishes stage III CAN from an earlier stage, preferably stage I and/or stage II.

The invention relates to a method for assessing the onset of chronic rejection of a transplanted organ in a subject, by obtaining a post-transplantation sample from the subject. The level of gene expression in the post-transplantation sample of a plurality of genes shown in Table 1 is determined. The level of gene expression of the at least one gene in the post-transplantation sample is compared with the level of gene expression of the same gene in a control sample. In one embodiment, a control includes biopsies from non transplanted healthy kidney or from transplanted kidney showing no sign of rejection. The up-regulation or down-regulation of at least one gene indicates that the subject is likely to experience transplant rejection, thereby assessing the onset of rejection of the transplanted organ in the subject. In one embodiment, the sample comprises cells obtained from the subject. In one embodiment, the sample is selected from the group consisting of: a graft biopsy; blood; serum; and urine. The method can be used to assess chronic transplant rejection and to determine the onset of rejection. The up-regulation or down-regulation of the plurality of genes in Table 1 provides a molecular signature which indicates that the subject is likely to experience chronic transplant rejection, *e.g.*, chronic/sclerosing allograph nephropathy. In a preferred embodiment, the stage of transplant rejection is grade I, grade II or grade III. The level of expression in the sample can be determined quantitatively.

A level of expression of the plurality of genes in the sample that differs from the control sample level of expression by a factor of at least about 1.5, 1.6, 1.7, 1.8, 1.9 or about 2.0, indicates that the subject is likely to experience chronic transplant rejection (refer to Table 4 for guidance on the genes from Table 1 that exhibit such changes in levels of expression). In the case of up regulation of expression, the changes seen in the genes is greater and therefore it is preferred that changes of levels of expression of at least 3.0 or more, preferably at least 3.5, 4, 5, 6 or more are observed in the marker genes of interest (refer to Table 4 for further guidance).

In one aspect, the invention provides a method for assessing the progression of rejection, e.g. chronic rejection, of a transplanted organ in a subject. A post-transplantation sample from a subject and the level of gene expression in the post-transplantation sample of a combination of a plurality of genes selected from the group of genes identified in Table 1 is determined. The pattern of gene expression of the at least one gene in the post-transplantation sample is compared with the pattern of gene expression of the same gene(s) in a control sample. Where a similarity in the expression pattern of the gene expression pattern of the combination of gene(s) in the post-transplantation sample compared to the expression pattern of the same combination of gene(s) in a control sample expression profile indicates a grade of transplant rejection, thereby assessing the progression of rejection of the transplanted organ in the subject. In one embodiment, the sample comprises cells obtained from the subject. In another embodiment, the sample is selected from the group consisting of: a graft biopsy; blood; serum; and urine. In one embodiment, the rejection is chronic/sclerosing allograft nephropathy. In one embodiment, the stage of transplant rejection is selected from the group consisting of: grade I; grade II; and grade III.

In another aspect, the invention pertains to a method of monitoring transplant rejection, e.g. chronic rejection, in a subject by taking as a baseline value the level of gene expression of a combination of a plurality of genes in a sample obtained from a transplanted subject who is known not to develop rejection. The level of gene expression corresponding to the combination of a plurality of genes is detected in a sample obtained from the subject post-transplantation. The first value is compared with the second value, wherein a first value lower or higher than the second value predicts that the transplanted subject is at risk of developing rejection, wherein the plurality of genes are defined in Table 1.

In another aspect, the invention pertains to a method of monitoring transplant rejection, e.g. chronic rejection, in a subject detecting a level of gene expression corresponding to a combination of a plurality of genes from a sample obtained from a donor subject at the day of transplantation. The level of gene expression corresponding to the plurality of genes is detected from a sample obtained from a recipient subject post-transplantation. The first level is compared with the second level, wherein a first level lower or higher than the second level predicts that the recipient subject is at risk of developing rejection; wherein the plurality of genes are as defined in Table 1.

In another aspect, the invention pertains a method for monitoring transplant rejection, e.g. chronic rejection, in a subject at risk thereof by obtaining a pre-administration sample from a transplanted subject prior to administration of a rejection inhibiting agent. The pattern of gene expression of a plurality of genes is detected in the pre-administration sample. One or more post-administration samples are obtained from the transplanted subject and the pattern of gene expression of a plurality of genes is detected in the post-administration sample or samples. The pattern of gene expression of the plurality of genes in the pre-administration sample is compared with the pattern of gene expression in the post-administration sample or samples, and the agent is adjusted accordingly, wherein the plurality of genes are defined in Table 1.

In another aspect, the invention pertains to a method for preventing, inhibiting, reducing or treating transplant rejection, e.g. chronic rejection, in a subject in need of such treatment comprising administering to the subject a compound that modulates the synthesis, expression or activity of one or more genes or gene products encoded thereof of genes as identified in Table 1, so that at least one symptom of rejection is ameliorated.

In another aspect, the invention pertains to a method for identifying agents for use in the prevention, inhibition, reduction or treatment of transplant rejection, e.g. chronic rejection, comprising monitoring the level of gene expression of one or more genes or gene products as identified in Table 1.

The pattern of gene expression can be assessed by detecting the presence of a protein encoded by the gene, for example by a reagent which specifically binds to the protein. The presence of the protein can be detected using a reagent which specifically binds to the protein. The pattern of gene expression can detected by techniques selected from the group consisting of Northern blot analysis, reverse transcription PCR and real time quantitative PCR.

Detecting the combination of the plurality of genes or expression products thereof as listed in Table 1 can be used as a biomarker for transplant rejection. Compound which modulate the synthesis, expression of activity of one or more genes as identified in Table 1, or an expression product thereof, can also be used for the preparation of a medicament for prevention or treatment of transplant rejection in a subject. The transplant rejection according to any method or use of the invention can be chronic/sclerosing allograft nephropathy and the gene(s) are selected from the group consisting of the genes identified in Table 1.

In another aspect, the invention pertains to a method of monitoring transplant rejection in a subject by taking as a baseline value the level of gene expression corresponding to a combination of a plurality of genes in a sample of a transplanted subject who is known not to develop rejection. The level of gene expression corresponding to the combination of the plurality of genes can be compared to the magnitude of gene expression in a sample obtained from a subject post-transplantation. The first value can be compared with the second value, wherein a first value lower or higher than the second value predicts that the transplanted subject is at risk of developing rejection, wherein the plurality of genes are as defined in Table 1.

In another embodiment of the invention, the method of transplant rejection monitoring may be performed by detecting a pattern of gene expression corresponding to a combination of a plurality of genes from a sample obtained from a donor subject at the day of transplantation and from a sample obtained from a recipient subject post-transplantation. The pattern of gene expression detected in the two samples can be compared. For example the magnitude of gene expression in the two samples can be compared. A similarity in the pattern of gene expression, e.g. in the magnitude of gene expression, predicts that the recipient subject is at risk of developing rejection.

In yet another aspect, the invention pertains to a method of monitoring transplant rejection in a subject by obtaining a pre-administration sample from a transplanted subject prior to administration of a rejection inhibiting agent; and detecting the pattern of gene expression, e.g. magnitude of gene expression, of a plurality of genes in the pre-administration sample. The pattern of gene expression, e.g. magnitude thereof, of the plurality of genes in the pre-administration sample can be compared with the pattern of gene expression in the post-administration sample or samples.

Thus, as a result of the work described herein, methods are now available to accurately quantitate marker gene expression in biopsy tissue, urine, peripheral blood mononuclear cells and other body fluids, and to correlate the magnitude of expression of these genes with rejection of chronic rejection of allografts, and in some embodiment to distinguish different stages of rejection.

The present invention also provides the use of a plurality of nucleic acid probes, each of which probes hybridizes specifically to a different gene in Table 1 as a biomarker for chronic/sclerosing allograft nephropathy

Preferably, the plurality of nucleic acid probes are capable of detecting expression of the SLC1A3, CD163, RDH12 and FLJ32569 genes.

### DETAILED DESCRIPTION

To further facilitate an understanding of the present invention, a number of terms and phrases are defined below:

The terms "down-regulation" or "down-regulated" are used interchangeably herein and refer to the decrease in the amount of a target gene or a target protein. The term "down-regulation" or "down-regulated" also refers to the decreases in processes or signal transduction cascades involving a target gene or a target protein.

The term "transplantation" as used herein refers to the process of taking a cell, tissue, or organ, called a "transplant" or "graft" from one subject and placing it or them into a (usually) different subject. The subject who provides the transplant is called the "donor" and the subject who received the transplant is called the "recipient". An organ, or graft, transplanted between two genetically different subjects of the same species is called an "allograft". A graft transplanted between subject s of different species is called a "xenograft".

The term "transplant rejection" as used herein is defined as functional and structural deterioration of the organ due to an active immune response expressed by the recipient, and independent of non-immunologic causes of organ dysfunction.

The term "acute rejection" as used herein refers to a rejection of the transplanted organ developing after the first 5-60 post-transplant days. It is generally a manifestation of cell-mediated immune injury. It is believed that both delayed hypersensitivity and cytotoxicity mechanisms are involved. The immune injury is directed against HLA, and possibly other cell-specific antigens expressed by the tubular epithelium and vascular endothelium.

The term "chronic rejection" as used herein refers to a rejection of the transplanted organ developing after the first 30-120 post-transplant days. The term "chronic rejection" also refers to a consequence of combined immunological injury (e.g. chronic rejection) and non-immunological damage (e.g. hypertensive nephrosclerosis, or nephrotoxicity of immunosuppressants like cyclosporine A), taking place month or years after transplantation and ultimately leading to fibrosis and sclerosis of the allograft, associated with progressive loss of kidney function.

The term "subject" as used herein refers to any living organism in which an immune response is elicited. The term subject includes, but is not limited to, humans, nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

A "gene" includes a polynucleotide containing at least one open reading frame that is capable of encoding a particular polypeptide or protein after being transcribed and translated. Any of the polynucleotide sequences described herein may be used to identify larger fragments or full-length coding sequences of the gene with which they are associated. Methods of isolating larger fragment sequences are known to those of skill in the art, some of which are described herein.

A "gene product" includes an amino acid (*e.g.*, peptide or polypeptide) generated when a gene is transcribed and translated.

The term "magnitude of expression" as used herein refers to quantifying marker gene transcript levels and comparing this quantity to the quantity of transcripts of a constitutively expressed gene. The term "level" or "magnitude of expression" means a "normalized, or standardized amount of gene expression". For example, the overall expression of all genes in cells varies (*i.e.*, is not constant). To accurately assess whether the detection of increased mRNA transcript is significant, it is preferable to "normalize" gene expression to accurately compare levels of expression between samples, *i.e.*, it is a base level against which gene expression is compared. Quantification of gene transcripts was accomplished using competitive reverse transcription polymerase chain reaction (RT-PCR) and the magnitude of gene expression was determined by calculating the ratio of the quantity of gene expression of each marker gene to the quantity of gene expression of the expressed gene.

The term "differentially expressed", as applied to a gene, includes the differential production of mRNA transcribed from a gene or a protein product encoded by the gene. A differentially expressed gene may be overexpressed or underexpressed as compared to the expression level of a normal or control cell. In one aspect, it includes a differential that is at least 1.1 times, 1.2 times. 1.3 times, 1.4 times, 1.5 times, 1.6 times, 1.7 times, 1.8 times, 1.9 times, at least 2 times, at least 3 times, at least 4 times, at least 5 times, at least 6 times, at least 7 times, at least 8 times, at least 9 times or at least 10 times higher or lower than the expression level detected in a control sample. In a preferred embodiment, the expression is higher than the control sample. The term "differentially expressed" also includes nucleotide sequences in a cell or tissue which are expressed where silent in a control cell or not expressed where expressed in a control cell.

The term "sample" as used herein refers to cells obtained from a biopsy. The term "sample" also refers to cells obtained from a fluid sample including, but not limited to, a sample of bronchoalveolar lavage fluid, a sample of bile, pleural fluid or peritoneal fluid, or any other fluid secreted or excreted by a normally or abnormally functioning allograft, or any other fluid resulting from exudation or transudation through an allograft or in anatomic proximity to an allograft, or any fluid in fluid communication with the allograft. A fluid test sample may also be obtained from essentially any body fluid including: blood (including peripheral blood), lymphatic fluid, sweat, peritoneal fluid, pleural fluid, bronchoalveolar lavage fluid, pericardial fluid, gastrointestinal juice, bile, urine, feces, tissue fluid or swelling fluid, joint fluid, cerebrospinal fluid, or any other named or unnamed fluid gathered from the anatomic area in proximity to the allograft or gathered from a fluid conduit in fluid communication with the allograft. A "post-transplantation fluid test sample" refers to a sample obtained from a subject after the transplantation has been performed.

Sequential samples can also be obtained from the subject and the quantification of immune activation gene markers determined as described herein, and the course of rejection can be followed over a period of time. In this case, for example, the baseline level (*e.g.*, magnitude) of gene expression of the immune activation marker genes is the level (*e.g.*, magnitude) of gene expression in a post-transplant sample taken after the transplant. For example, an initial sample or samples can be taken within the nonrejection period, for example, within one week of transplantation and the level (*e.g.*, magnitude) of expression of marker genes in these samples can be compared with the level (*e.g.*, magnitude) of expression of the genes in samples taken after one week. In one embodiment, the samples are taken on weeks 6, 12 and 24 post-transplantation

The term "biopsy" as used herein refers to a specimen obtained by removing tissue from living patients for diagnostic examination. The term includes aspiration biopsies, brush biopsies, chorionic villus biopsies, endoscopic biopsies, excision biopsies, needle biopsies (specimens obtained by removal by aspiration through an appropriate needle or trocar that pierces the skin, or the external surface of an organ, and into the underlying tissue to be examined), open biopsies, punch biopsies (trephine), shave biopsies, sponge biopsies, and wedge biopsies. In one embodiment, a fine needle aspiration biopsy is used. In another embodiment, a minicore needle biopsy is used. A conventional percutaneous core needle biopsy can also be used.

The term "up-regulation" or "up-regulated" are used interchangeably herein and refer to the increase or elevation in the amount of a target gene or a target protein. The term "up-regulation" or "up-regulated" also refers to the increase or elevation of processes or signal transduction cascades involving a target gene or a target protein.

The term "down-regulation" or "down-regulated" are used interchangeably herein and refer to the decrease or elevation in the amount of a target gene or a target protein. The term "down-regulation" or "down-regulated" also refers to the decrease or reduction of processes or signal transduction cascades involving a target gene or a target protein.

A "probe set" as used herein refers to a group of nucleic acids that may be used to detect two or more genes. Detection may be, for example, through amplification as in PCR and RT-PCR, or through hybridization, as on a microarray, or through selective destruction and protection, as in assays based on the selective enzymatic degradation of single or double stranded nucleic acids. Probes in a probe set may be labeled with one or more fluorescent, radioactive or other detectable moieties (including enzymes). Probes may be any size so long as the probe is sufficiently large to selectively detect the desired gene. A probe set may be in solution, as would be typical for multiplex PCR, or a probe set may be adhered to a solid surface, as in an array or microarray. It is well known that compounds such as PNAs may be used instead of nucleic acids to hybridize to genes. In addition, probes may contain rare or unnatural nucleic acids such as inosine.

The terms "polynucleotide" and "oligonucleotide" are used interchangeably, and include polymeric forms of nucleotides of any length, either deoxyribonucleotides or ribonucleotides, or analogs thereof. Polynucleotides may have any three-dimensional structure, and may perform any function, known or unknown. The following are non-limiting examples of polynucleotides: a gene or gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The term also includes both double- and single-stranded molecules. Unless otherwise specified or required, any embodiment of this invention that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for guanine when the polynucleotide is RNA. This, the term "polynucleotide sequence" is the alphabetical representation of a polynucleotide molecule. This alphabetical representation can be inputted into databases in a computer having a central processing unit and used for bioinformatics applications such as functional genomics and homology searching.

The term "cDNAs" includes complementary DNA, that is mRNA molecules present in a cell or organism made into cDNA with an enzyme such as reverse transcriptase. A "cDNA library" includes a collection of mRNA molecules present in a cell or organism, converted into cDNA molecules with the enzyme reverse transcriptase, then inserted into "vectors" (other DNA molecules that can continue to replicate after addition of foreign DNA). Exemplary vectors for libraries include bacteriophage, viruses that infect bacteria (*e.g.*, lambda phage). The library can then be probed for the specific cDNA (and thus mRNA) of interest.

A "primer" includes a short polynucleotide, generally with a free 3'-OH group that binds to a target or "template" present in a sample of interest by hybridizing with the target, and thereafter promoting polymerization of a polynucleotide complementary to the target. A "polymerase chain reaction" ("PCR") is a reaction in which replicate copies are made of a target polynucleotide using a "pair of primers" or "set. of primers" consisting of "upstream" and a "downstream" primer, and a catalyst of polymerization, such as a DNA polymerase, and typically a thermally-stable polymerase enzyme. Methods for PCR are well known in the art, and are taught, for example, in MacPherson *et al.,* IRL Press at Oxford University Press (1991)). All processes of producing replicate copies of a polynucleotide, such as PCR or gene cloning, are collectively referred to herein as "replication". A primer can also be used as a probe in hybridization reactions, such as Southern or Northern blot analyses (see, *e.g.*, Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, N.Y., 1989).

The term "polypeptide" includes a compound of two or more subunit amino acids, amino acid analogs, or peptidomimetics. The subunits may be linked by peptide bonds. In another embodiment, the subunit may be linked by other bonds, *e.g.*, ester, ether, etc. As used herein the term "amino acid" includes either natural and/or unnatural or synthetic amino acids, including glycine and both the D or L optical isomers, and amino acid analogs and peptidomimetics. A peptide of three or more amino acids is commonly referred to as an oligopeptide. Peptide chains of greater than three or more amino acids are referred to as a polypeptide or a protein.

The term "hybridization" includes a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Hybridization reactions can be performed under conditions of different "stringency". The stringency of a hybridization reaction includes the difficulty with which any two nucleic acid molecules will hybridize to one another. Under stringent conditions, nucleic acid molecules at least 60%, 65%, 70%, 75% identical to each other remain hybridized to each other, whereas molecules with low percent identity cannot remain hybridized. A preferred, non-limiting example of highly stringent hybridization conditions are hybridization in 6Xsodium chloride/sodium citrate (SSC) at about 45°C, followed by one or more washes in 0.2XSSC, 0.1 % SDS at 50°C, preferably at 55°C, more preferably at 60°C, and even more preferably at 65°C.

When hybridization occurs in an antiparallel configuration between two single-stranded polynucleotides, the reaction is called "annealing" and those polynucleotides are described as "complementary". A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to hydrogen bond with each other, according to generally accepted base-pairing rules.

As used herein, the term "marker" includes a polynucleotide or polypeptide molecule which is present or increased in quantity or activity in subjects at risk for organ rejection. The relative change in quantity or activity of the marker is correlated with the incidence or risk of incidence of rejection.

As used herein, the term "panel of markers" includes a group of markers, the quantity or activity of each member of which is correlated with the incidence or risk of incidence of organ rejection. In certain embodiments, a panel of markers may include only those markers which are either increased in quantity or activity in subjects at risk for organ rejection. A panel of markers preferably comprises at least 4 markers, such as at least 5, 6, 7, 8, 9 or 10 markers, e.g. at least 15 markers. Preferred genes are described elsewhere.

As used herein, the term "differential expression profile" means a profile containing values for each relevant marker which form the basis of the profile, the values being derived by comparing the magnitude of expression in the test sample material of each marker of interest (whether at the RNA or protein level, but preferably at the RNA level), with the magnitude of expression of the same marker of interest in a control sample (or with a previously derived control value) and calculating e.g. the fold variation or percentage variation. A non-limiting example of an differential expression profile is given in Table 4 (each of the last three columns). A differential expression profile preferably comprises values for at least 4 markers, such as at least 5, 6, 7, 8, 9 or 10 markers, e.g. at least 15 markers.

As used herein, the term "reference differential expression profile" is a differential expression profile which is known to be representative of one or more stages of CAN and which can be used as a basis for comparison with a differential expression profile from a test sample to determine whether the test sample profile is sufficiently similar to the reference profile to enable classification of the test sample. A reference differential expression profile may be (and typically is) derived by pooling results from expression analysis of samples where the stage of CAN is known.

### Biomarkers of Chronic Rejection

This present invention provides biomarkers for the progression of renal fibrosis and chronic inflammation useful as diagnostic and/or prognostic tools for the prevention and treatment of CAN. The invention is based, in part, on the finding that select genes are modulated in chronic transplant dysfunction, *e.g.*, CAN, the extent of which variations is different between the various stages/grades of CAN. Advances in highly parallel, automated DNA hybridization techniques combined with the growing wealth of human gene sequence information have made it feasible to simultaneously analyze expression levels for thousands of genes. Methods such as the gene-by-gene quantitative RT-PCR are highly accurate but relatively labor intensive. While it is possible to analyze the expression of thousands of genes using quantitative PCR, the effort and expense would be enormous. Instead, as an example of large scale analysis, an entire population of mRNAs may be converted to cDNA and hybridized to an ordered array of probes that represent anywhere from ten to ten thousand or more genes. The relative amount of cDNA that hybridizes to each of these probes is a measure of the expression level of the corresponding gene. The data may then be statistically analyzed to reveal informative patterns of gene expression. Indeed, early diagnosis of renal allograft rejection and new prognostic biomarkers are important minimize and personalize immunosuppression. In addition to histopathological differential diagnosis, gene expression profiling significantly improves disease classification by defining a "molecular signature."

Several previous studies have successfully applied a transcriptomic approach to distinguish different classes of kidney transplants. However, the heterogeneity of microarray platforms and various data analysis methods complicates the identification of robust signatures of CAN.

To address this issue, data analysis was performed on gene expression profiles of renal protocol biopsies from patients with stable graft function and patients who had been diagnosed with varying grades of CAN. As presented in Example I, this study identified the intersection of multiple gene expression signatures from different microarray datasets to derive descriptors (*i.e.*, gene markers; biomarkers) which can accurately classify tissue with CAN. That is, the present invention relates to the identification of genes, which are modulated *(i.e.,* up-regulated or down-regulated) during rejection, in particular during CAN progression. A highly statistically significant correlation has been found between the expression of one or more biomarker gene(s) and CAN, thereby providing a "molecular signature" for transplant rejection (*e.g.*, CAN). These biomarker genes and their expression products can be used in the management, prognosis and treatment of patients at risk of transplant rejection as they are useful to identify organs that are likely to undergo rejection and/or to determine the grade of disease (*e.g.*, CAN) progression.

The data disclosed herein demonstrate that in actual clinical situations, and in the absence of molecular manipulations of gene expression, combinations of genes are indicative of CAN. In one embodiment, the combination of biomarker genes/probes that form a molecular signature after tissue transplantation are those shown below in Table 1.

**Table 1: CAN specific expression signature of 33 probe sets/32 genes**

| **Rank** | **Affymetrix probe set** | **Gene symbol** | **RefSeq (NCBI)** | **Gene description** |
|---|---|---|---|---|
| 1 | 202800_at | SLC1A3 | NM_004172 | solute carrier family 1 (glial high affinity glutamate transporter), member 3 |
| 2 | 203645_s_at | CD163 | NM_004244, | CD163 molecule (hemoglobin-haptoglobin |
| 3 | 215049_x_at | | NM_203416 | receptor) |
| 4 | 219840_s_at | TCL6 | NM_012468, NM_014418 | T-cell leukemia/lymphoma |
| 5 | 231994_at | CHDH | (AJ272267) | choline dehydrogenase |
| 6 | 239929_at | | NM_152491 (carboxypeptidase | hypothetical protein FLJ32569 activity) |
| 7 | 242274_x_at | SLC25A42 | NM_178526 | solute carrier family 25, member 42 |
| 8 | 232271_at | HNF4G | NM_004133 | hepatocyte nuclear factor 4, gamma |
| 9 | 203222_s_at | TLE1 | NM_005077 Drosophila | transducin-like enhancer of split 1 homolog, |
| 10 | 89977_at | | NM_017888 process) | hypothetical protein FLJ20581 (metabolic |
| 11 | 242998_at | RDH12 | NM_152443 | retinol dehydrogenase 12 (all-trans/9-cis/11-cis) |
| 12 | 235964_x_at | | (AA603344) | cDNA clone IMAGE:1117747 |
| 13 | 234219_at | | (AK024998) | cDNA FLJ21345 fis |
| 14 | 230179_at | | (AK127555) | cDNA FLJ45648 fis |
| 15 | 204438_at | MRC1 | NM_002438 | mannose receptor, C type 1 |
| | | MRC1L1 | NM_001009567 | mannose receptor, C type 1-like 1 |
| 16 | 201761_at | MTHFD2 | NM_006636. | methylenetetrahydrofolate dehydrogenase (NADP⁺ dependent) 2, methenyltetrahydrofolate cyclohydrolase, nuclear gene encoding mitochondrial protein |
| | | | NM_001040409 | |
| 17 | 219090_at | SLC24A3 | NM_020689 (sodium/potassium/calcium | solute carrier family 24 exchanger), member 3 |
| 18 | 219260_s_at | C17orf81 | NM_015362, | chromosome 17 open reading frame 81 (DERP6) |
| | | | NM_203413-15 | |
| 19 | 239983_at | SLC30A8 | NM_173851 member | solute carrier family 30 (zinc transporter), 8 |
| 20 | 201041_s_at | DUSP1 | NM_004417 | dual specificity phosphatase 1 |
| 21 | 213519_s_at | LAMA2 | NM_000426, | laminin, alpha 2 (merosin, congenital muscular dystrophy) |
| | | | NM_001079823 | |
| 22 | 205278_at | GAD1 | NM_000817 GAD67 | glutamate decarboxylase 1, transcript variant |
| 23 | 225662_at | ZAK | NM_133646 | sterile alpha motif and leucine zipper containing kinase AZK, transcript variant 2 |
| 24 | 239161_at | FDX1 | NM_004109 | ferredoxin 1, nuclear gene encoding mitochondrial protein |
| 25 | 217762_s_at | RAB31 | NM_006868 | RAB31, member RAS oncogene family |
| 26 | 230716_at | | XM_379432 | hypothetical protein LOC285733 |
| 27 | 236442_at | DPF3 | NM_012074 | D4, zinc and double PHD fingers, family 3 |
| 28 | 207095_at | SLC10A2 | NM_000452 cotransporter family), | solute carrier family 10 (sodium/bile acid member 2 |
| 29 | 226142_at | GLIPR1 | NM_006851 | GLI pathogenesis-related 1 |
| 30 | 213817_at | | (AL049435) | cDNA DKFZp586B0220 |
| 31 | 224357_s_at | MS4A4A | NM_024021, NM_148975 | membrane-spanning 4-domains, subfamily A, member 4 |
| | | | | |
| 32 | 223582_at | GPR98 | NM_032119, NR_003149 | G protein-coupled receptor 98 |
| | | | | |
| 33 | 229554_at | LUM | NM_002345 | lumican, member of the small leucine-rich proteoglycan (SLRP) family |

Accordingly, in one aspect the invention relates to using a recognition signature comprising one or more of the genes shown in Table 1 to indicate transplant rejection, in particular CAN rejection of a transplanted organ, preferably at least 5, 10, 15, 20 or 25 of the genes of Table 1 (the rankings shown can be used as guidance for those genes that it is most preferred to include in the assessment).

The 33 probe sets are also listed in Table 4 where the fold change in expression relative to control is indicated for CAN I, II and III. The table is sorted on the results for CAN III. Preferred genes whose expression profile is analysed in the methods of the invention are (identified by the Affymetrix probe set indicated in Table 1 from which the gene name can be derived): 203645_s_at, 215049_x_at, 229554_at, 235964_x_at, 202800 (upregulated) and 242998_at, 219840_s_at, 239929_at, 223582_at and 205278_at (downregulated). Particularly preferred are the genes corresponding to probe sets 202800_at, 215049_x_at and 203645_s_at (i.e. SLC1A3 and CD163), and the genes corresponding to probe sets 219840_s_at and 239929_at (i.e. RDH12 and FLJ32569). These preferred genes/probe sets can be particularly useful for the detection of CAN III and/or distinguishing CAN III from CAN I/CAN II and/or AR.

### Clinical Features of CAN

Chronic transplant dysfunction is a phenomenon in solid organ transplants displaying a gradual deterioration of graft function months to years after transplantation, eventually leading to graft failure, and which is accompanied by characteristic histological features. Clinically, chronic allograft nephropathy in kidney grafts (i.e., CAN) manifests itself as a slowly progressive decline in glomerular filtration rate, usually in conjunction with proteinuria and arterial hypertension.

The cardinal histomorphologic feature of CAN in all parenchymal allografts is fibroproliferative endarteritis. The vascular lesion affects the whole length of the arteries in a patchy pattern. There is concentric myointimal proliferation resulting in fibrous thickening and the characteristic 'onion skin' appearance of the intima in small arteries. Other findings include endothelial swelling, foam cell accumulation, disruption of the internal elastic lamina, hyalinosis and medial thickening, and presence of subendothelial T-lymphocytes and macrophages. In addition, a persistent focal perivascular inflammation is often seen.

In addition to vascular changes, kidneys undergoing CAN also show interstitial fibrosis, tubular atrophy, and glumerulopathy. Chronic transplant glumerolopathy - duplication of the capillary walls and mesangial matrix increase - has been identified as a highly specific feature of kidneys with CAN. Less specific lesions are glomerular ischemic collapse, tubular atrophy, and interstitial fibrosis. Furthermore, peritubular capillary basement splitting and laminations are associated with late decline of graft function. The criteria for histological diagnosis of CAN in kidney allografts are internationally standardized in the Banff 97 scheme for Renal Allograft Pathology. Table 2 summarizes the Banff 97 criteria for chronic/sclerosing allograft nephropathy (CAN) (Racusen et al., 1999, Kidney Int. 55(2):713-23).

| Table 2 - Banff 97 criteria for CAN | |
|---|---|
| Grade | Histopathological Findings |
| I - mild | Mild interstitial fibrosis and tubular atrophy without (a) or with (b) specific changes suggesting chronic rejection |
| II - moderate | Moderate interstitial fibrosis and tubular atrophy (a) or (b) |
| III - severe | Severe interstitial fibrosis and tubular atrophy and tubular loss (a) or (b) |

For Banff 97, an "adequate" specimen is defined as a biopsy with 10 or more glumeruli and at least two arteries. Two working hypotheses are proposed to understand the process of CAN. The first and probably the most important set of risk factors have been lumped under the designation of "alloantigen-dependent", immunological or rejection-related factors. Among these, late onset and increased number of acute rejection episodes; younger recipient age; male-to-female sex mismatch; a primary diagnosis of autoimmune hepatitis or biliary disease; baseline immunosuppression and non-caucasian recipient race have all been associated with an increased risk of developing chronic rejection. More specifically, (a) histoincompatibility: long-term graft survival appear to be strongly correlated with their degree of histocompatibility matching between donor and recipient; (b) Acute rejections: onset, frequency, and severity of acute rejection episodes are independent risk factors of CAN. Acute rejection is the most consistently identified risk factor for the occurrence of CAN; (c) Suboptimal immunosuppression due to too low maintenance dose of cyclosporine or non-compliance; and (d) Anti-donor specific antibodies: many studies have shown that following transplantation, the majority of patients produce antibodies. The second set of risk factors are referred to as "non-alloantigen-dependent" or "non-immunological" risk factors that also contribute to the development of chronic rejection include advanced donor age, pre-existing atherosclerosis in the donor organ, and prolonged cold ischemic time. Non-alloimmune responses to disease and injury, such as ischemia, can cause or aggravate CAN. More specifically, (a) recurrence of the original disease, such as glomerulonephritis; (b) consequence of the transplantation surgical injury; (c) duration of ischemia: intimal hyperplasia correlates with duration of ischemia; (d) kidney grafts from cadavers versus those from living related and unrelated donors; (e) viral infections: CMV infection directly affects intercellular adhesion molecules such as ICAM-1; (f) hyperlipidemia; (g) hypertension; (h) age; (i) gender: the onset of transplant arterosclerosis was earlier in male than in female; (j) race; and (k) the amount of functional tissue - reduced number of nephrons and hyperfiltration.

### Limitations to Current Clinical Approaches for CAN Diagnosis

The differentiation of the diagnosis of rejection, *e.g.*, CAN, from other etiologies for graft dysfunction and institution of effective therapy is a complex process because: (a) the percutaneous core needle biopsy of grafts, the best of available current tools to diagnose rejection is performed usually after the "fact", *i.e.*, graft dysfunction and graft damage (irreversible in some instances) are already present, (b) the morphological analysis of the graft provides modest clues with respect to the potential for reversal of a given rejection episode, and minimal clues regarding the likelihood of recurrence ("rebound"), and (c) the mechanistic basis of the rejection phenomenon, a prerequisite for the design of therapeutic strategies, is poorly defined by current diagnostic indices, including morphologic features of rejection.

The diagnosis of, for example, renal allograft rejection is made usually by the development of graft dysfunction (*e.g.*, an increase in the concentration of serum creatinine) and morphologic evidence of graft injury in areas of the graft also manifesting mononuclear cell infiltration. Two caveats apply, however, to the use of abnormal renal function as an indicator of the rejection process: first, deterioration in renal function is not always available as a clinical clue to diagnose rejection since many of the cadaveric renal grafts suffer from acute (reversible) renal failure in the immediate post-transplantation period due to injury from harvesting and *ex vivo* preservation procedures. Second, even when immediately unimpaired renal function is present, graft dysfunction might develop due to a non-immunologic cause, such as immunosuppressive therapy itself.

For example, cyclosporine (CsA) nephrotoxicity, a complication that is not readily identified solely on the basis of plasma/blood concentrations of CsA, is a common complication. The clinical importance of distinguishing rejection from CsA nephrotoxicity cannot be overemphasized since the therapeutic strategies are diametrically opposite: escalation of immunosuppressants for rejection, and reduction of CsA dosage for nephrotoxicity.

The invention is based, in part, on the finding that increased or decreased expression of one or more genes and/or the encoded proteins can be reliably associated with certain graft rejection states. Thus, as a result of the data described herein, methods are now available for the rapid and reliable diagnosis of acute and chronic rejection, even in cases where allograft biopsies show only mild cellular infiltrates. Described herein is an analysis of genes that are modulated (*e.g.*, up-regulated or down-regulated) simultaneously and which provide a molecular signature to accurately detect transplant rejection and/or grade the severity or progression of transplant rejection.

The invention further provides classic molecular methods and large scale methods for measuring expression of suitable biomarker genes. The methods described herein are particularly useful for detecting chronic transplant rejection and preferably early chronic transplant rejection. In one embodiment, the chronic transplant rejection is the result of CAN. Most typically, the subject (*i.e.*, the recipient of a transplant) is a mammal, such as a human. The transplanted organ can include any transplantable organ or tissue, for example kidney, heart, lung, liver, pancreas, bone, bone marrow, bowel, nerve, stem cells (or stem cell-derived cells), tissue component and tissue composite. In a preferred embodiment, the transplant is a kidney transplant.

The methods described herein are useful to assess the efficacy of anti-rejection therapy. Such methods involve comparing the pre-administration level (*e.g.*, magnitude) of the transcripts of the biomarker genes to the post-administration level (*e.g.*, magnitude) of the transcripts of the same genes, where a post-administration level (*e.g.*, magnitude) of the transcripts of the genes that is less than the pre-administration level (*e.g.*, magnitude) of the transcripts of the same genes indicates the efficacy of the anti-rejection therapy. Any candidates for prevention and/or treatment of transplant rejection, (such as drugs, antibodies, or other forms of rejection or prevention) can be screened by comparison of level (*e.g.*, magnitude) of biomarker expression before and after exposure to the candidate. In addition, valuable information can be gathered in this manner to aid in the determination of future clinical management of the subject upon whose biological material the assessment is being performed. The assessment can be performed using a sample from the subject, using the methods described herein for determining the level (*e.g.*, magnitude) of gene expression of the biomarker genes. Analysis can further comprise detection of an infectious agent.

It is to be appreciated that the present invention provides methods wherein the level (*e.g.*, magnitude) of expression of CAN biomarkers are measured and that from these measurements a pattern of expression of CAN biomarkers can be derived which is also useful in select methods of the present invention.

### Detecting Gene Expression

In certain aspects of the present invention, the level (e.g., magnitude) of expression is determined for one or more biomarker genes in sample obtained from a subject. The sample can comprise cells obtained from the subject, such as from a graft biopsy. Other samples include, but are not limited to fluid samples such as blood, plasma, serum, lymph, CSF, cystic fluid, ascites, urine, stool and bile. The sample may also be obtained from bronchoalveolar lavage fluid, pleural fluid or peritoneal fluid, or any other fluid secreted or excreted by a normally or abnormally functioning allograft, or any other fluid resulting from exudation or transudation through an allograft or in anatomic proximity to an allograft, or any fluid in fluid communication with the allograft.

Individuals who have had a kidney transplant will typically present for testing as a result of experiencing one or more symptoms that may indicate the onset of rejection of the transplanted organ. This may occur at any stage after the transplant has been performed.

Many different methods are known in the art for measuring gene expression. Classical methods include quantitative RT-PCR, Northern blots and ribonuclease protection assays. Certain examples described herein use competitive reverse transcription (RT)-PCR to measure the level (*e.g.*, magnitude) of expression of marker genes. Such methods may be used to examine expression of subject genes as well as entire gene clusters. However, as the number of genes to be examined increases, the time and expense may become cumbersome.

Large scale detection methods allow faster, less expensive analysis of the expression levels of many genes simultaneously. Such methods typically involve an ordered array of probes affixed to a solid substrate. Each probe is capable of hybridizing to a different set of nucleic acids. In one method, probes are generated by amplifying or synthesizing a substantial portion of the coding regions of various genes of interest. These genes are then spotted onto a solid support. Then, mRNA samples are obtained, converted to cDNA, amplified and labeled (usually with a fluorescence label). The labeled cDNAs are then applied to the array, and cDNAs hybridize to their respective probes in a manner that is linearly related to their concentration. Detection of the label allows measurement of the amount of each cDNA adhered to the array.

Many methods for performing such DNA array experiments are well known in the art. Exemplary methods are described below but are not intended to be limiting. Microarrays are known in the art and consist of a surface to which probes that correspond in sequence to gene products (*e.g.*, cDNAs, mRNAs, oligonucleotides) are bound at known positions. In one embodiment, the microarray is an array (*i.e.*, a matrix) in which each position represents a discrete binding site for a product encoded by a gene (*e.g.*, a protein or RNA), and in which binding sites are present for products of most or almost all of the genes in the organism's genome. In a preferred embodiment, the "binding site" (hereinafter, "site") is a nucleic acid or nucleic acid derivative to which a particular cognate cDNA can specifically hybridize. The nucleic acid or derivative of the binding site can be, *e.g.*, a synthetic oligomer, a full-length cDNA, a less-than full length cDNA, or a gene fragment.

Usually the microarray will have binding sites corresponding to at least 100 genes and more preferably, 500, 1000, 4000 or more. In certain embodiments, the most preferred arrays will have about 98-100% of the genes of a particular organism represented. In other embodiments, customized microarrays that have binding sites corresponding to fewer, specifically selected genes can be used. In certain embodiments, customized microarrays comprise binding sites for fewer than 4000, fewer than 1000, fewer than 200 or fewer than 50 genes, and comprise binding sites for at least 2, preferably at least 3, 4, 5 or more genes of any of the biomarkers of Table 1. Preferably, the microarray has binding sites for genes relevant to testing and confirming a biological network model of interest.

The nucleic acids to be contacted with the microarray may be prepared in a variety of ways. Methods for preparing total and poly(A)+ RNA are well known and are described generally in Sambrook *et a*/*.*, *supra.* Labeled cDNA is prepared from mRNA by oligo dT-primed or random-primed reverse transcription, both of which are well known in the art (see *e.g.*, Klug and Berger, 1987, Methods Enzymol. 152: 316-325). Reverse transcription may be carried out in the presence of a dNTP conjugated to a detectable label, most preferably a fluorescently labeled dNTP. Alternatively, isolated mRNA can be converted to labeled antisense RNA synthesized by *in vitro* transcription of double-stranded cDNA in the presence of labeled dNTPs (Lockhart et al., 1996, Nature Biotech. 14: 1675). The cDNAs or RNAs can be synthesized in the absence of detectable label and may be labeled subsequently, *e.g.*, by incorporating biotinylated dNTPs or rNTP, or some similar means (*e.g.*, photo-cross-linking a psoralen derivative of biotin to RNAs), followed by addition of labeled streptavidin (*e.g.*, phycoerythrin-conjugated streptavidin) or the equivalent.

When fluorescent labels are used, many suitable fluorophores are known, including fluorescein, lissamine, phycoerythrin, rhodamine (Perkin Elmer Cetus), Cy2, Cy3, Cy3.5, Cy5, Cy5.5, Cy7, FluorX (Amersham) and others (see, *e.g.*, Kricka, 1992, Academic Press San Diego, Calif.).

In another embodiment, a label other than a fluorescent label is used. For example, a radioactive label, or a pair of radioactive labels with distinct emission spectra, can be used. However, use of radioisotopes is a less-preferred embodiment.

Nucleic acid hybridization and wash conditions are chosen so that the population of labeled nucleic acids will specifically hybridize to appropriate, complementary nucleic acids affixed to the matrix. As used herein, one polynucleotide sequence is considered complementary to another when, if the shorter of the polynucleotides is less than or equal to 25 bases, there are no mismatches using standard base-pairing rules or, if the shorter of the polynucleotides is longer than 25 bases, there is no more than a 5% mismatch.

Optimal hybridization conditions will depend on the length (*e.g.*, oligomer versus polynucleotide greater than 200 bases) and type (*e.g.*, RNA, DNA, PNA) of labeled nucleic acids and immobilized polynucleotide or oligonucleotide. General parameters for specific (*i.e.*, stringent) hybridization conditions for nucleic acids are described in Sambrook *et al.*, *supra*, and in Ausubel et al., 1987, Current Protocols in Molecular Biology, Greene Publishing and Wiley-Interscience, New York, which is incorporated in its entirety for all purposes. Non-specific binding of the labeled nucleic acids to the array can be decreased by treating the array with a large quantity of non-specific DNA - a so-called "blocking" step.

When fluorescently labeled probes are used, the fluorescence emissions at each site of a transcript array can be, preferably, detected by scanning confocal laser microscopy. When two fluorophores are used, a separate scan, using the appropriate excitation line, is carried out for each of the two fluorophores used. Alternatively, a laser can be used that allows simultaneous specimen illumination at wavelengths specific to the two fluorophores and emissions from the two fluorophores can be analyzed simultaneously (see Shalon et al., 1996, Genome Research 6:639-645). In a preferred embodiment, the arrays are scanned with a laser fluorescent scanner with a computer controlled X-Y stage and a microscope objective. Sequential excitation of the two fluorophores is achieved with a multiline, mixed gas laser and the emitted light is split by wavelength and detected with two photomultiplier tubes. Fluorescence laser scanning devices are described in Schena et al., 1996, Genome Res. 6:639-645 and in other references cited herein. Alternatively, the fiber-optic bundle described by Ferguson et al., 1996, Nature Biotech. 14:1681-1684, may be used to monitor mRNA abundance levels at a large number of sites simultaneously. Fluorescent microarray scanners are commercially available from Affymetrix, Packard BioChip Technologies, BioRobotics and many other suppliers.

Signals are recorded, quantitated and analyzed using a variety of computer software. In one embodiment the scanned image is despeckled using a graphics program (*e.g.*, Hijaak Graphics Suite) and then analyzed using an image gridding program that creates a spreadsheet of the average hybridization at each wavelength at each site. If necessary, an experimentally determined correction for "cross talk" (or overlap) between the channels for the two fluors may be made. For any particular hybridization site on the transcript array, a ratio of the emission of the two fluorophores is preferably calculated. The ratio is independent of the absolute expression level of the cognate gene, but is useful for genes whose expression is significantly modulated by drug administration, gene deletion, or any other tested event.

In one embodiment, transcript arrays reflecting the transcriptional state of a cell of interest are made by hybridizing a mixture of two differently labeled sets of cDNAs to the microarray. One cell is a cell of interest while the other is used as a standardizing control. The relative hybridization of each cell's cDNA to the microarray then reflects the relative expression of each gene in the two cells.

It is not always necessary to measure the levels of expression in a control cells at the same time as the cell of interest. Measurements can be compared against a standard set of controls. Typically, the standard controls include the levels of expression of a house keeping gene, such as actin, so that the results for the sample of interest can be adjusted to those of the control to take into account differences in assay conditions, background levels etc.

The end result is typically a set of values of the relative expression of the marker genes in the sample of interest compared to a control (e.g. up or down and the magnitude of change expressed in, for example, percentage terms or as a multiple). This set of relative values is termed a "differential expression profile" and is generally used as the basis for subsequent diagnosis/classification. The differential expression profile is typically then compared to a reference differential expression profile which characterizes one or more stages/grades of CAN. For example a single reference differential expression profile may be used which is indicative of any stage of CAN. Alternatively, or in addition, a reference differential expression profile which is specific for one or more stages, but not all stages, or CAN e.g. a profile for stage III and a different profile for stages I and II. Since some variation is to be expected, statistical analysis can be used to determine whether a profile of interest is sufficiently close to the reference profile for a statistically significant match to be found.

In some embodiments, expression levels of biomarkers in different samples and conditions may be compared using a variety of statistical methods. A variety of statistical methods are available to assess the degree of relatedness in expression patterns of different genes. The statistical methods may be broken into two related portions: metrics for determining the relatedness of the expression pattern of one or more gene, and clustering methods, for organizing and classifying expression data based on a suitable metric (Sherlock, 2000, Curr. Opin. Immunol. 12:201-205; Butte et al., 2000, Pacific Symposium on Biocomputing, Hawaii, World Scientific, p.418-29).

In one embodiment, Pearson correlation may be used as a metric. In brief, for a given gene, each data point of gene expression level defines a vector describing the deviation of the gene expression from the overall mean of gene expression level for that gene across all conditions. Each gene's expression pattern can then be viewed as a series of positive and negative vectors. A Pearson correlation coefficient can then be calculated by comparing the vectors of each gene to each other. An example of such a method is described in Eisen *et al.* (1998, *supra*). Pearson correlation coefficients account for the direction of the vectors, but not the magnitudes.

In another embodiment, Euclidean distance measurements may be used as a metric. In these methods, vectors are calculated for each gene in each condition and compared on the basis of the absolute distance in multidimensional space between the points described by the vectors for the gene. In another embodiment, both Euclidean distance and Correlation coefficient were used in the clustering.

In a further embodiment, the relatedness of gene expression patterns may be determined by entropic calculations (Butte *et al.* 2000, *supra*). Entropy is calculated for each gene's expression pattern. The calculated entropy for two genes is then compared to determine the mutual information. Mutual information is calculated by subtracting the entropy of the joint gene expression patterns from the entropy calculated for each gene individually. The more different two gene expression patterns are, the higher the joint entropy will be and the lower the calculated mutual information. Therefore, high mutual information indicates a non-random relatedness between the two expression patterns.

In another embodiment, agglomerative clustering methods may be used to identify gene clusters. In one embodiment, Pearson correlation coefficients or Euclidean metrics are determined for each gene and then used as a basis for forming a dendrogram. In one example, genes were scanned for pairs of genes with the closest correlation coefficient. These genes are then placed on two branches of a dendrogram connected by a node, with the distance between the depth of the branches proportional to the degree of correlation. This process continues, progressively adding branches to the tree. Ultimately a tree is formed in which genes connected by short branches represent clusters, while genes connected by longer branches represent genes that are not clustered together. The points in multidimensional space by Euclidean metrics may also be used to generate dendrograms.

In yet another embodiment, divisive clustering methods may be used. For example, vectors are assigned to each gene's expression pattern, and two random vectors are generated. Each gene is then assigned to one of the two random vectors on the basis of probability of matching that vector. The random vectors are iteratively recalculated to generate two centroids that split the genes into two groups. This split forms the major branch at the bottom of a dendrogram. Each group is then further split in the same manner, ultimately yielding a fully branched dendrogram.

In a further embodiment, self-organizing maps (SOM) may be used to generate clusters. In general, the gene expression patterns are plotted in n-dimensional space, using a metric such as the Euclidean metrics described above. A grid of centroids is then placed onto the n-dimensional space and the centroids are allowed to migrate towards clusters of points, representing clusters of gene expression. Finally the centroids represent a gene expression pattern that is a sort of average of a gene cluster. In certain embodiments, SOM may be used to generate centroids, and the genes clustered at each centroid may be further represented by a dendrogram. An exemplary method is described in Tamayo et al., 1999, PNAS 96:2907-12. Once centroids are formed, correlation must be evaluated by one of the methods described *supra.*

In another embodiment, PLSDA, OPLS and OSC multivariate analyses may be used as a means of classification.

In another aspect, the invention provides probe sets. Preferred probe sets are designed to detect expression of one or more genes and provide information about the status of a graft. Preferred probe sets of the invention comprise probes that are useful for the detection of at least two genes belonging to any of the biomarker genes of Table 1. Probe sets of the invention comprise probes useful for the detection of no more than 10,000 gene transcripts, and preferred probe sets will comprise probes useful for the detection of fewer than 4000, fewer than 1000, fewer than 200, fewer than 100, fewer than 90, fewer than 80, fewer than 70, fewer than 60, fewer than 50, fewer than 40, fewer than 30, fewer than 20, fewer than 10 gene transcripts. The probe sets of the invention are targeted at the detection of gene transcripts that are informative about transplant status. Probe sets of the invention may also comprise a large or small number of probes that detect gene transcripts that are not informative about transplant status. In preferred embodiments, probe sets of the invention are affixed to a solid substrate to form an array of probes. It is anticipated that probe sets may also be useful for multiplex PCR. The probes of probe sets may be nucleic acids (*e.g.*, DNA, RNA, chemically modified forms of DNA and RNA), or PNA, or any other polymeric compound capable of specifically interacting with the desired nucleic acid sequences.

Computer readable media comprising a biomarker(s) of the present invention is also provided. As used herein, "computer readable media" includes a medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. The skilled artisan will readily appreciate how any of the presently known computer readable mediums can be used to create a manufacture comprising computer readable medium having recorded thereon a biomarker of the present invention.

As used herein, "recorded" includes a process for storing information on computer readable medium. Those skilled in the art can readily adopt any of the presently known methods for recording information on computer readable medium to generate manufactures comprising the biomarkers of the present invention.

A variety of data processor programs and formats can be used to store the biomarker information of the present invention on computer readable medium. For example, the nucleic acid sequence corresponding to the biomarkers can be represented in a word processing text file, formatted in commercially-available software such as WordPerfect and MicroSoft Word, or represented in the form of an ASCII file, stored in a database application, such as DB2, Sybase, Oracle, or the like. Any number of dataprocessor structuring formats (*e.g.*, text file or database) may be adapted in order to obtain computer readable medium having recorded thereon the biomarkers of the present invention.

By providing the biomarkers of the invention in computer readable form, one can routinely access the biomarker sequence information for a variety of purposes. For example, one skilled in the art can use the nucleotide or amino acid sequences of the invention in computer-readable form to compare a target sequence or target structural motif with the sequence information stored within the data storage means. Search means are used to identify fragments or regions of the sequences of the invention which match a particular target sequence or target motif.

The invention also includes an array comprising a biomarker(s) of the present invention. The array can be used to assay expression of one or more genes in the array. In one embodiment, the array can be used to assay gene expression in a tissue to ascertain tissue specificity of genes in the array. In this manner, up to about 8600 genes can be simultaneously assayed for expression. This allows a profile to be developed showing a battery of genes specifically expressed in one or more tissues.

In addition to such qualitative determination, the invention allows the quantitation of gene expression. Thus, not only tissue specificity, but also the level of expression of a battery of genes in the tissue is ascertainable. Thus, genes can be grouped on the basis of their tissue expression per se and level of expression in that tissue. This is useful, for example, in ascertaining the relationship of gene expression between or among tissues. Thus, one tissue can be perturbed and the effect on gene expression in a second tissue can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development and differentiation, disease progression, *in vitro* processes, such a cellular transformation and senescence, autonomic neural and neurological processes, such as, for example, pain and appetite, and cognitive functions, such as learning or memory.

The array is also useful for ascertaining the effect of the expression of a gene on the expression of other genes in the same cell or in different cells. This provides, for example, for a selection of alternate molecular targets for therapeutic intervention if the ultimate or downstream target cannot be regulated.

The array is also useful for ascertaining differential expression patterns of one or more genes in normal and diseased cells. This provides a battery of genes that could serve as a molecular target for diagnosis or therapeutic intervention.

### Proteins

It is further anticipated that increased levels of certain proteins may also provide diagnostic information about transplants. In certain embodiments, one or more proteins encoded by genes of Table 1 may be detected, and elevated or decreased protein levels may be used to diagnose graft rejection. In a preferred embodiment, protein levels are detected in a post-transplant fluid sample, and in a particularly preferred embodiment, the fluid sample is peripheral blood or urine. In another preferred embodiment, protein levels are detected in a graft biopsy.

In view of this specification, methods for detecting proteins are well known in the art. Examples of such methods include Western blotting, enzyme-linked immunosorbent assays (ELISAs), one- and two-dimensional electrophoresis, mass spectroscopy and detection of enzymatic activity. Suitable antibodies may include polyclonal, monoclonal, fragments (such as Fab fragments), single chain antibodies and other forms of specific binding molecules.

### Predictive Medicine

The present invention pertains to the field of predictive medicine in which diagnostic assays, prognostic assays, pharmacogenetics and monitoring clinical trials are used for prognostic (predictive) purposes to thereby diagnose and treat a subject prophylactically. Accordingly, one aspect of the present invention relates to diagnostic assays for determining biomarker protein and/or nucleic acid expression from a sample (*e.g.*, blood, serum, cells, tissue) to thereby determine whether a subject is likely to reject a transplant.

Another aspect of the invention pertains to monitoring the influence of agents (*e.g.*, drugs, compounds) on the expression or activity of biomarker in clinical trials as described in further detail in the following sections.

An exemplary method for detecting the presence or absence of biomarker protein or genes of the invention in a sample involves obtaining a sample from a test subject and contacting the sample with a compound or an agent capable of detecting the protein or nucleic acid (*e.g.*, mRNA, genomic DNA) that encodes the biomarker protein such that the presence of the biomarker protein or nucleic acid is detected in the sample. A preferred agent for detecting mRNA or genomic DNA corresponding to a biomarker gene or protein of the invention is a labeled nucleic acid probe capable of hybridizing to a mRNA or genomic DNA of the invention. Suitable probes for use in the diagnostic assays of the invention are described herein.

A preferred agent for detecting biomarker protein is an antibody capable of binding to biomarker protein, preferably an antibody with a detectable label. Antibodies can be polyclonal, or more preferably, monoclonal. An intact antibody, or a fragment thereof (*e.g.*, Fab or F(ab')2) can be used. The term "labeled", with regard to the probe or antibody, is intended to encompass direct labeling of the probe or antibody by coupling (*i.e.*, physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "sample " is intended to include tissues, cells and biological fluids isolated from a subject, as well as tissues, cells and fluids present within a subject. That is, the detection method of the invention can be used to detect biomarker mRNA, protein, or genomic DNA in a sample *in vitro* as well as *in vivo.* For example, *in vitro* techniques for detection of biomarker mRNA include Northern hybridizations and *in situ* hybridizations. *In vitro* techniques for detection of biomarker protein include enzyme linked immunosorbent assays (ELISAs), Western blots, immunoprecipitations and immunofluorescence. *In vitro* techniques for detection of biomarker genomic DNA include Southern hybridizations. Furthermore, *in vivo* techniques for detection of biomarker protein include introducing, into a subject, a labeled anti-biomarker antibody. For example, the antibody can be labeled with a radioactive biomarker whose presence and location in a subject can be detected by standard imaging techniques.

In one embodiment, the sample contains protein molecules from the test subject. Alternatively, the sample can contain mRNA molecules from the test subject or genomic DNA molecules from the test subject. A preferred sample is a serum sample isolated by conventional means from a subject.

The methods further involve obtaining a control sample (*e.g.*, biopsies from non transplanted healthy kidney or from transplanted healthy kidney showing no sign of rejection) from a control subject, contacting the control sample with a compound or agent capable of detecting biomarker protein, mRNA, or genomic DNA, such that the presence of biomarker protein, mRNA or genomic DNA is detected in the sample, and comparing the presence of biomarker protein, mRNA or genomic DNA in the control sample with the presence of biomarker protein, mRNA or genomic DNA in the test sample.

The invention also encompasses kits for detecting the presence of biomarker in a sample. For example, the kit can comprise a labeled compound or agent capable of detecting biomarker protein or mRNA in a sample; means for determining the amount of biomarker in the sample; and means for comparing the amount of biomarker in the sample with a standard. The compound or agent can be packaged in a suitable container. The kit can further comprise instructions for using the kit to detect biomarker protein or nucleic acid.

The diagnostic methods described herein can furthermore be utilized to identify subjects having or at risk of developing a disease or disorder associated with aberrant biomarker expression or activity. As used herein, the term "aberrant" includes a biomarker expression or activity which deviates from the wild type biomarker expression or activity. Aberrant expression or activity includes increased or decreased expression or activity, as well as expression or activity which does not follow the wild type developmental pattern of expression or the subcellular pattern of expression. For example, aberrant biomarker expression or activity is intended to include the cases in which a mutation in the biomarker gene causes the biomarker gene to be under-expressed or overexpressed and situations in which such mutations result in a non-functional biomarker protein or a protein which does not function in a wild-type fashion, *e.g.*, a protein which does not interact with a biomarker ligand or one which interacts with a non-biomarker protein ligand.

Furthermore, the prognostic assays described herein can be used to determine whether a subject can be administered an agent (*e.g.*, an agonist, antagonist, peptidomimetic, protein, peptide, nucleic acid, small molecule, or other drug candidate) to reduce the risk of rejection, *e.g.*, cyclospsorin. Thus, the present invention provides methods for determining whether a subject can be effectively treated with an agent for a disorder associated with increased gene expression or activity of the combination of genes in Table 1.

Monitoring the influence of agents (*e.g.*, drugs) on the expression or activity of a genes can be applied not only in basic drug screening, but also in clinical trials. For example, the effectiveness of an agent determined by a screening assay as described herein to increase gene expression, protein levels, or up-regulate activity, can be monitored in clinical trials of subjects exhibiting by examining the molecular signature and any changes in the molecular signature during treatment with an agent.

For example, and not by way of limitation, genes and their encoded proteins that are modulated in cells by treatment with an agent (*e.g.*, compound, drug or small molecule) which modulates gene activity can be identified. In a clinical trial, cells can be isolated and RNA prepared and analyzed for the levels of expression of genes implicated associated with rejection. The levels of gene expression (*e.g.*, a gene expression pattern) can be quantified by northern blot analysis or RT-PCR, as described herein, or alternatively by measuring the amount of protein produced, by one of the methods as described herein. In this way, the gene expression pattern can serve as a molecular signature, indicative of the physiological response of the cells to the agent. Accordingly, this response state may be determined before, and at various points during treatment of the subject with the agent.

In a preferred embodiment, the present invention provides a method for monitoring the effectiveness of treatment of a subject with an agent (*e.g.*, an agonist, antagonist, peptidomimetic, protein, peptide, nucleic acid, small molecule, or other drug candidate identified by the screening assays described herein) including the steps of (i) obtaining a pre-administration sample from a subject prior to administration of the agent; (ii) detecting the level of expression of a gene or combination of genes, the protein encoded by the genes, mRNA, or genomic DNA in the pre-administration sample; (iii) obtaining one or more post-administration samples from the subject; (iv) detecting the level of expression or activity of the biomarker protein, mRNA, or genomic DNA in the post-administration samples; (v) comparing the level of expression or activity of the biomarker protein, mRNA, or genomic DNA in the pre-administration sample with the a gene or combination of genes, the protein encoded by the genes, mRNA, or genomic DNA in the post administration sample or samples; and (vi) altering the administration of the agent to the subject accordingly. For example, increased administration of the agent may be desirable to decrease the expression or activity of the genes to lower levels, *i.e.*, to increase the effectiveness of the agent to protect against transplant rejection. Alternatively, decreased administration of the agent may be desirable to decrease expression or activity of biomarker to lower levels than detected, *i.e.*, to decrease the effectiveness of the agent, *e.g.*, to avoid toxicity. According to such an embodiment, gene expression or activity may be used as an indicator of the effectiveness of an agent, even in the absence of an observable phenotypic response.

The present invention provides for both prophylactic and therapeutic methods for preventing transplant rejection. With regards to both prophylactic and therapeutic methods of treatment, such treatments may be specifically tailored or modified, based on knowledge obtained from the field of pharmacogenomics. "Pharmacogenomics", as used herein, includes the application of genomics technologies such as gene sequencing, statistical genetics, and gene expression analysis to drugs in clinical development and on the market. More specifically, the term refers the study of how a subject's genes determine his or her response to a drug (*e.g.*, a subject's "drug response phenotype", or "drug response genotype"). Thus, another aspect of the invention provides methods for tailoring a subject's prophylactic or therapeutic treatment with either the biomarker molecules of the present invention or biomarker modulators according to that subject's drug response genotype. Pharmacogenomics allows a clinician or physician to target prophylactic or therapeutic treatments to subjects who will most benefit from the treatment and to avoid treatment of subjects who will experience toxic drug-related side effects.

In one aspect, the invention provides a method for preventing transplant rejection in a subject, associated with increased biomarker expression or activity, by administering to the subject a compound or agent which modulates biomarker expression. Examples of such compounds or agents are e.g., compounds or agents having immunosuppressive properties, such as those used in transplantation (*e.g.*, a calcineurin inhibitor, cyclosporin A or FK 506); a mTOR inhibitor (*e.g.*, rapamycin, 40-O-(2-hydroxyethyl)-rapamycin, CCI779, ABT578, AP23573, biolimus-7 or biolimus-9); an ascomycin having immuno-suppressive properties (*e.g.*, ABT-281, ASM981, etc.); corticosteroids; cyclophosphamide; azathioprene; methotrexate; leflunomide; mizoribine; mycophenolic acid or salt; mycophenolate mofetil; 15-deoxyspergualine or an immunosuppressive homologue, analogue or derivative thereof, a PKC inhibitor (*e.g.*, as disclosed in WO 02/38561 or WO 03/82859, the compound of Example 56 or 70); a JAK3 kinase inhibitor (*e.g.*, N-benzyl-3,4-dihydroxy-benzylidene-cyanoacetamide a-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxy quinazoline] WHI-P97, KRX-211, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, *e.g.*, mono-citrate (also called CP-690,550), or a compound as disclosed in WO 04/052359 or WO 05/066156); a S1 P receptor agonist or modulator (*e.g.*, FTY720 optionally phosphorylated or an analog thereof, *e.g.*, 2-amino-2-[4-(3-benzyloxyphenylthio)-2-chlorophenyl]ethyl-1,3-propanediol optionally phosphorylated or 1-{4-[1-(4-cyclohexyl-3-trifluoromethyl-benzyloxyimino)-ethyl]-2-ethyl-benzyl}-azetidine-3-carboxylic acid or its pharmaceutically acceptable salts); immunosuppressive monoclonal antibodies (e.g., monoclonal antibodies to leukocyte receptors, e.g., MHC, CD2, CD3, CD4, CD7, CD8, CD25, CD28, CD40, CD45, CD52, CD58, CD80, CD86 or their ligands); other immunomodulatory compounds (*e.g.*, a recombinant binding molecule having at least a portion of the extracellular domain of CTLA4 or a mutant thereof, *e.g.*, an at least extracellular portion of CTLA4 or a mutant thereof joined to a non-CTLA4 protein sequence, *e.g.*, CTLA4Ig (for ex. designated ATCC 68629) or a mutant thereof, *e.g.*, LEA29Y); adhesion molecule inhibitors (*e.g.*, LFA-1 antagonists, ICAM-1 or -3 antagonists, VCAM-4 antagonists or VLA-4 antagonists). These compounds or agents may also be used in combination.

Another aspect of the invention pertains to methods of modulating biomarker protein expression or activity for therapeutic purposes. Accordingly, in an exemplary embodiment, the modulatory method of the invention involves contacting a cell with a biomarker protein or agent that modulates one or more of the activities of a biomarker proteins activity associated with the cell. An agent that modulates biomarker protein activity can be an agent as described herein, such as a nucleic acid or a protein, a naturally-occurring target molecule of a biomarker protein (*e.g.*, a biomarker protein substrate), a biomarker protein antibody, a biomarker protein agonist or antagonist, a peptidomimetic of a biomarker protein agonist or antagonist, or other small molecule. In one embodiment, the agent stimulates one or more biomarker protein activities. Examples of such stimulatory agents include active biomarker protein and a nucleic acid molecule encoding biomarker protein that has been introduced into the cell. In another embodiment, the agent inhibits one or more biomarker protein activities. Examples of such inhibitory agents include antisense biomarker protein nucleic acid molecules, anti-biomarker protein antibodies, and biomarker protein inhibitors. These modulatory methods can be performed *in vitro* (*e.g.,* by culturing the cell with the agent) or, alternatively, *in vivo* (*e.g.,* by administering the agent to a subject). As such, the present invention provides methods of treating a subject afflicted with a disease or disorder characterized by aberrant expression or activity of a biomarker protein or nucleic acid molecule. In one embodiment, the method involves administering an agent (*e.g.*, an agent identified by a screening assay described herein), or combination of agents that modulates (*e.g.*, up-regulates or down-regulates) biomarker protein expression or activity. In another embodiment, the method involves administering a biomarker protein or nucleic acid molecule as therapy to compensate for reduced or aberrant biomarker protein expression or activity.

Stimulation of biomarker protein activity is desirable in situations in which biomarker protein is abnormally down-regulated and/or in which increased biomarker protein activity is likely to have a beneficial effect. For example, stimulation of biomarker protein activity is desirable in situations in which a biomarker is down-regulated and/or in which increased biomarker protein activity is likely to have a beneficial effect. Likewise, inhibition of biomarker protein activity is desirable in situations in which biomarker protein is abnormally up-regulated and/or in which decreased biomarker protein activity is likely to have a beneficial effect.

The biomarker protein and nucleic acid molecules of the present invention, as well as agents, or modulators which have a stimulatory or inhibitory effect on biomarker protein activity (*e.g.*, biomarker gene expression), as identified by a screening assay described herein, can be administered to subjects to treat (prophylactically or therapeutically) biomarker-associated disorders (*e.g.*, prostate cancer) associated with aberrant biomarker protein activity. In conjunction with such treatment, pharmacogenomics (*i.e.*, the study of the relationship between a subject's genotype and that subject's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a biomarker molecule or biomarker modulator as well as tailoring the dosage and/or therapeutic regimen of treatment with a biomarker molecule or biomarker modulator.

One pharmacogenomics approach to identifying genes that predict drug response, known as "a genome-wide association", relies primarily on a high-resolution map of the human genome consisting of already known gene-related biomarkers (*e.g.*, a "bi-allelic" gene biomarker map which consists of 60,000-100,000 polymorphic or variable sites on the human genome, each of which has two variants). Such a high-resolution genetic map can be compared to a map of the genome of each of a statistically significant number of subjects taking part in a Phase II/III drug trial to identify biomarkers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, subjects can be grouped into genetic categories depending on a particular pattern of SNPs in their subject genome. In such a manner, treatment regimens can be tailored to groups of genetically similar subjects, taking into account traits that may be common among such genetically similar subjects.

Alternatively, a method termed the "candidate gene approach", can be utilized to identify genes that predict drug response. According to this method, if a gene that encodes a drugs target is known (*e.g.*, a biomarker protein of the present invention), all common variants of that gene can be fairly easily identified in the population and it can be determined if having one version of the gene *versus* another is associated with a particular drug response.

The invention is based, in part, on the observation that increased or decreased expression of many different genes and/or the encoded proteins is associated with certain graft rejection states. As a result of the data described herein, methods are now available for the rapid and reliable diagnosis of acute and chronic rejection, even in cases where allograft biopsies show only mild cellular infiltrates. Described herein for the first time is an analysis of genes that are up-regulated and/or down-regulated simultaneously and which provide a molecular signature to accurately detect transplant rejection.

In addition, the invention is partly based on the observation that genes are expressed as gene clusters - groups of genes, often functionally related, that have substantially related expression profiles under certain circumstances Accordingly, the invention provides clusters of genes, the expression of the members of which is correlated with graft rejection. The invention further provides classic molecular methods and large scale methods for measuring expression of suitable marker genes.

The methods described herein are particularly useful for detecting chronic transplant rejection. Most typically, the subject (*i.e.*, the recipient of a transplant) is a mammal, such as a human. The transplanted organ can include any transplantable organ or tissue, for example kidney, heart, lung, liver, pancreas, bone, bone marrow, bowel, nerve, stem cells (or stem cell-derived cells), tissue component and tissue composite. In a preferred embodiment, the transplant is a kidney transplant.

Information generated from more than one of the above pharmacogenomics approaches can be used to determine appropriate dosage and treatment regimens for prophylactic or therapeutic treatment an subject. This knowledge, when applied to dosing or drug selection, can avoid adverse reactions or therapeutic failure and thus enhance therapeutic or prophylactic efficiency when treating a subject with a marker molecule or marker modulator, such as a modulator identified by one of the exemplary screening assays described herein.

This invention is further illustrated by the following examples which should not be construed as limiting. The contents of all references, patents and published patent applications cited throughout this application, are incorporated herein by reference.

### EXAMPLE: Molecular Signature Analysis (MSA) for Chronic Rejection.

Over the past years, high-density oligonucleotide or cDNA microarrays data have been used to classify and predict allograft rejection and treatment outcome (Weintraub and Sarwal, 2006, Transplant International 19:775-881. New data analysis methods allow now to extract the biological information at pathway levels, and in close correlation with clinical parameters, therefore redefining disease pathology via transcriptome changes. In this study, we exploited genomics to identify differentially expressed genes in renal diagnostic biopsies from a cohort of 49 patients displaying different types and grades of AR and CR. Prediction analysis of microarray (PAM) algorithm (Tibshirani et al., 2002, PNAS 99: 6567-72), and support vector machine (SVM) algorithm were selected to identify Banff 97 molecular signatures.

### Materials and Methods

### Patients

All patients at Hôpital Tenon, Paris, undergoing a diagnostic renal allografts biopsy (February 2003 until September 2004) and consenting were included in the study. In addition, few patients from Hôpital Bicêtre, Paris, and Hôpital Pellegrin, Bordeaux, were recruited. In total, 75 diagnostic renal core biopsies were collected in RNAlater (Ambion, Austin, TX). Part of each biopsy was separately sampled and processed for histological staining and analysis. Another 19 control samples were taken from nephrectomy specimens of patients suffering from solid renal cancer using non affected renal cortex at maximal distance from circumscribed malignancies.

Clinical description of the patients and histopathological assessment of biopsies (Banff 97 classification) were collected. For CAN, different disease severity scores were determined for cg, glomerulopathy, ci, interstitial fibrosis, ct, tubular atrophy, cv, vascular atrophy/intimal thickening, and ah, arterial hyaline thickening. Table 3 displays the most important clinical parameters and the overall Banff 97 classification for all patients finally included in this analysis after stringent quality control of extracted RNA and microarray hybridizations.

**Table 3: Demographic and clinical characteristics of the five groups of the Paris biopsies for clinical indications according to histological analysis**

| **Parameter** | **CAN** | **CAN + AR** | **AR** | **Borderline** | **NR** |
|---|---|---|---|---|---|
| Number of biopsies | 22 | 7 | 8 | 3 | 7 |
| Number of patients* | 22 | 6 | 8 | 2 | 7 |
| Recipient age [years] | 46.9 ±12.2^{#} | 41.7 ±7.6 | 44.9 ±10.9 | 34.6 ±10.2 | 39.4 ±8.7# |
| Recipient gender [n, % | 15 | 2 (33%) | 6 (75%) | 1 (50%) | 6 (86%) |
| male] | (68.2%) | | | | |
| Donor age [years] | 43.4 ±17.1^{#} | 39.0 ±19.8 | 36.3 ±8.3^{#} | 46.5 ±0.7 | 45.2 ±15.4 |
| # HLA mismatches | 2.9±1.3^{#} | 2.7 ±2.1^{#} | 2.8±1.6^{#} | 3.5±0.7 | 1.8 ±1.5^{#} |
| # historic AR episodes [% | 36.4 | 83.3 | 75 | 100 | 14.3 |
| patients with≥1] | | | | | |
| Time of biopsy [months post tx] | 83.2 ±64.8 | 52.1 ±48.3 | 28.1 ±51.1 | 3.4 ±4.9 | 25.1 ±51.4 |
| Number of patients with | | | 3 0 1 0 | | 1 |
| CNI toxicity (histology) | | | | | |
| Number of patients on a | 2 | 0 | 1 | 0 | 0 |
| CNI-free regimen | | | | | |
| Serum creatinine [µMol] | 268.8 | 461.1 | 253.0 | 223.0 | 160.0 |
| | ±208.5 | ±317.3 | ±109.3 | ±40.7^{#} | ±44.4 |

| | | | | | |
|---|---|---|---|---|---|
| Abbreviations: CAN = chronic allograft nephropathy, AR = acute rejection, NR = no rejection (but not stable). *5 patients had two sequential biopsies; # some values unknown All patients were on standard CNI triple regimen: 68% cyclosporine and 32% tacrolimus based, mainly combined with mycophenolate and corticosteroids. Ethnicity: 60% Caucasian, 18% African, 22% other origin. Included are all samples used for signature generation (see supplementary material table S1 for individual data). | | | | | |

### Preparation of RNA

Total RNA was extracted by RNeasy according to the manufacturer's protocol (Qiagen, Hilden, Germany). RNA was quantified by a NanoDrop ND-1000 spectrophotometer (NanDrop Technologies, Wilmington, DE) and quality controlled by a 2100 Bioanalyzer (Agilent Technologies, Santa Clara, CA). Only biopsies resulting in high quality total RNA were further analyzed. 50 ng of total RNA were subjected to Affymetrix 2-cycle cDNA amplification, fluorescent labeling, and hybridization to the Human Genome U133 Plus 2.0 Array containing ∼54,625 probe sets for >47,000 different human transcripts (Affymetrix, Santa Clara, CA).

### Microarray analysis

A single weighted mean expression level for each gene along with a p-value indicating reliable transcript detection was derived using Microarray Suite 5.0 software (MAS5, Affymetrix). Data were scaled from each array (target intensity of 150). For further analysis, the cell intensity (CEL) files were subjected to the Robust Multichip Analysis (RMA) normalization. Several quality control measures on each array were assessed, including review of the scanned image for significant artifacts, background and noise measurements that differ significantly from other chips, average of present and absent calls. Furthermore, arrays failing two out of the three following quality control measures were excluded from the study: the 3' to 5' ratio of the intensities for glyceraldehyde-3-phosphate dehydrogenase (ratio <4 considered suitable), average of calls (>40% of present calls was acceptable), scaling factor (>2 or <0.5) were excluded from the analysis.

4 samples (3 nephrectomized and 1 AR) were excluded based on the quality control filters described above. In addition, 3 nephrectomized samples were considered as biological outliers based on their global gene expression profile. In total, 63 data sets were obtained matching quality control criteria (Table 1).

Analysis of the raw Genechip^{®} data was performed using software GeneSpring GX7.3 (Agilent Technologies). Genes differentially expressed among different samples classes (e.g., control versus AR) were identified using a one-way ANOVA (p<0.05), with or without a false discovery rate ≤5% (FDR, Benjamini and Hochberg) and additional cutoff based on 2-fold change expression.

In the absence of a broad consensus regarding class prediction algorithm, we decided to show results coming of prediction analysis of microarrays (PAM) (Wieczorek et al., 2006, American Journal of Transplantation 6:1285-96), which show consistent and robust results in our hands (PAM version 1.28 available from BioConductor or www-stat.stanford.edu/∼tibs/ PAM/ Rdist/). A 10-fold cross validation was performed and the overall misclassification rate was reported. Different significance-level cutoffs were chosen to find the lowest cross-validation misclassification rate with the minimum number of genes. Support Vector Machine (SVM) algorithm (GeneSpring GX 7.3) was also used for class prediction and show very similar results compared to PAM in most of the cases.

### Molecular signature analysis for chronic rejection

Our initial aim was to define a molecular signature/classifier that could differentiate between the different grades of CR and control samples. To generate the molecular signature, we analyzed a total of 35 biopsies, 13 biopsies from the control nephrectomized patients, 4 CAN grade I, 10 CAN grade II and 8 CAN grade III samples. The PAM algorithm identified a set of 33 genes able to discriminate the different groups with the minimal cross validation error rate of 0.12, i.e., the different grades of CR are classified with 88% accuracy. Using 2 unsupervised clustering methods, based on the expression profile of these 33 genes, we were able to separate patients into groups corresponding to histological grades. Among this geneset, 15 are gradually up regulated from grade I to III while the 18 remaining genes are down-regulated (Table 1). While grade I and III are clearly separated from each other, grade II samples present an intermediate heterogeneous stage, which might correspond to an evolved active disease status.

In order to assess the performance of the molecular classifier, we tested it on two independent samples sets. First, we took the opportunity to analyzed gene expression data from an internal non-human primate (NHP) model of chronic rejection. Interestingly a PCA analysis with the 33 genes defined in human CAN, clearly discriminate chronic rejected from normal monkey transplanted kidneys. The PAM analysis predicted with 100% accuracy the 2 group of biopsies. In a second step, we tested 3 new CAN grade I samples, coming from a second sampling, and 7 CAN samples that were not included in the initial analysis (3 grade II and 4 grade III samples which also present levels of acute rejection (AR+CR samples)). These 10 new samples were used as a validation set, while the initial set of 35 biopsies was used as a training set. A class prediction SVM algorithm with the 33 genes signature was able to correctly predict all the new samples except one grade II, which was classified as grade I, displaying an overall accuracy of 89%. This 2 independent results demonstrate the relevance and robustness of the molecular signature identified for CAN, regardless the low number of biopsies included in this study.

This study demonstrates the possibility to use molecular signature analysis to differentiate classes of allograft rejection. For the first time, this work showed that a molecular signature could differentiate biopsies according to gradual severity of CAN (I, II, III). The list of 33 probesets/genes identified in this analysis, correctly classified different CAN grades with 90% accuracy in human and 100% accuracy in a NHP study of CAV. It is difficult to achieve a lower misclassification rate because of the inherent ambiguity of the visual histopathological diagnosis. Differences between CAN grade I and II are slight and prone to subjective weighting by individual pathologists. This trend is well reflected by gene expression. For example, while grade III samples are easily separated, several samples could be classified as either grade I or II. In this respect, grade I and grade II samples are often differentially classified by the PAM and SVM algorithms (data not shown). Global gene expression suggests that grade II biopsy samples undergo a very active and progressing stage of CAN rendering this patient group heterogeneous. Indeed, it is surprising to observe that grade II samples display a significant enrichment of genes associated with immune function and inflammation at a similar level than AR, but much more than grade I or III samples. However, CAN III may represent a late stage of tissue sclerosis with a declining transcriptional activity with respect to these two conditions. Early differential diagnosis will be important for intervention to lessen CAN before the putatively irreversible grade III manifests, taken into account that 25% of patients demonstrate grade II CAN one-year post-transplantation).

Pathway analysis of CAN revealed that biological processes in early CAN behave differently from later grades with a slight but significant increase in energy and metabolism pathway genes that are decreasing with disease progression. This phenomenon might be explained by compensatory renal hypertrophy well known in diabetic nephropathy. Here, injury of the kidney is compensated early by hypertrophy and proliferation of mesangial and parenchymal cells leading to increased tubular function. During early stages of CAN, it is tempting to hypothesize that this compensatory mechanism occurs and is reflected at the transcriptional level. Compared to AR and grade II CAN, the vast majority of identified genes changed significantly in progressed human CAN III. Here, they are associated with TGF and Wnt signaling pathways, indicative of interstitial fibrosis progression and development/activation of myofibroblasts under chronic inflammatory condition.

Those skilled in the art will recognize, or be able to ascertain using no more than routine experimentation, many equivalents to the specific embodiments of the invention described herein. Such equivalents are intended to be encompassed by the following claims.

The various features and embodiments of the present invention, referred to in individual sections above apply, as appropriate, to other sections, mutatis mutandis. Consequently features specified in one section may be combined with features specified in other sections, as appropriate.

All publications mentioned in the above specification are herein incorporated by reference. All of the methods disclosed and claimed in this specification can be made and executed without undue experimentation in light of the present disclosure. While the methods of this invention have been described in terms of preferred embodiments, it will be apparent to those of skill in the art that variations may be applied to the methods and in the steps or in the sequence of steps of the method described herein without departing from the concept, spirit and scope of the invention

**Table 4 - Expression Analysis**

| ***Affy Ids*** | ***I*** | ***II*** | ***III*** | ***control*** | *ECI.vs **control*** | ***FC II.vs control*** | ***FC III vs control*** |
|---|---|---|---|---|---|---|---|
| 203645_s_at | 324.46 | 1198.72 | 2270.94 | 261.13 | 1.24 | 4.59 | 8.70 |
| 215049_x_at | 417.40 | 1634.22 | 3044.82 | 360.08 | 1.16 | 4.54 | 8.46 |
| 229554_at | 150.30 | 416.06 | 643.58 | 99.32 | 1.51 | 4.19 | 6.48 |
| 235964_x_at | 555.11 | 1307.39 | 1977.18 | 319.81 | 1.74 | 4.09 | 6.18 |
| 202800_at | 153.24 | 333.99 | 712.59 | 160.57 | 0.95 | 2.08 | 4.44 |
| 213817_at | 35.47 | 81.51 | 178.22 | 40.71 | 0.87 | 2.00 | 4.38 |
| 204438_at | 602.89 | 1269.26 | 2011.43 | 469.11 | 1.29 | 2.71 | 4.29 |
| 201761_at | 227.91 | 446.65 | 821.61 | 200.31 | 1.14 | 2.23 | 4.10 |
| 226142_at | 43.90 | 116.01 | 170.77 | 42.40 | 1.04 | 2.74 | 4.03 |
| 225662_at | 398.36 | 643.62 | 1351.06 | 409.82 | -1.03 | 1.57 | 3.30 |
| 217762_s_at | 282.89 | 553.52 | 859.79 | 265.55 | 1.07 | 2.08 | 3.24 |
| 213519_s_at | 191.45 | 346.06 | 536.19 | 178.84 | 1.07 | 1.93 | 3.00 |
| 230179_at | 117.70 | 199.64 | 347.95 | 116.33 | 1.01 | 1.72 | 2.99 |
| 203222_s_at | 45.28 | 79.35 | 106.35 | 46.83 | -1.03 | 1.69 | 2.27 |
| 219090_at | 119.98 | 172.57 | 233.84 | 115.76 | 1.04 | 1.49 | 2.02 |
| 224357_s_at | 352.36 | 444.14 | 545.73 | 387.84 | -1.10 | 1.15 | 1.41 |
| 219260_s_at | 395.30 | 170.32 | 240.65 | 232.58 | 1.70 | -1.37 | 1.03 |
| 239161_at | 676.92 | 411.71 | 362.41 | 427.19 | 1.58 | -1.04 | -1.18 |
| 236442_at | 250.21 | 137.61 | 134.10 | 174.29 | 1.44 | -1.27 | -1.30 |
| 234219_at | 614.77 | 355.49 | 173.82 | 226.71 | 2.71 | 1.57 | -1.30 |
| 242274_x_at | 59.62 | 34.61 | 25.25 | 35.70 | 1.67 | -1.03 | -1.41 |
| 231994_at | 854.58 | 619.25 | 444.97 | 644.19 | 1.33 | -1.04 | -1.45 |
| 89977_at | 674.84 | 421.99 | 280.13 | 434.88 | 1.55 | -1.03 | -1.55 |
| 230716_at | 339.70 | 175.67 | 112.57 | 210.21 | 1.62 | -1.20 | -1.87 |
| 232271_at | 780.42 | 402.48 | 245.12 | 469.04 | 1.66 | -1.17 | -1.91 |
| 239983_at | 338.56 | 123.38 | 97.93 | 191.63 | 1.77 | -1.55 | -1.96 |
| 201041_s_at | 3670.49 | 1935.86 | 5397.73 | 11170.30 | -3.04 | -5.77 | -2.07 |
| 207095_at | 805.75 | 285.76 | 230.38 | 506.75 | 1.59 | -1.77 | -2.20 |
| 242998_at | 708.77 | 256.05 | 174.83 | 402.16 | 1.76 | -1.57 | -2.30 |
| 219840_s_at | 341.20 | 155.14 | 80.98 | 195.63 | 1.74 | -1.26 | -2.42 |
| 239929_at | 483.71 | 104.66 | 74.90 | 225.73 | 2.14 | -2.16 | -3.01 |
| 223582_at | 715.99 | 221.92 | 126.52 | 480.31 | 1.49 | -2.16 | -3.80 |
| 205278_at | 220.05 | 60.19 | 80.03 | 359.59 | -1.63 | -5.97 | -4.49 |

## Claims

1. A method for assessing the onset of a chronic rejection of a transplanted kidney in a subject, comprising the steps of:
(a) determining the level of expression in a post-transplantation sample from a subject of a combination of a plurality of genes selected from the group consisting of the genes identified in Table 1;
(b) comparing the level of gene expression of said plurality of genes in the post-transplantation sample with the magnitude of gene expression of the same genes in a control sample to generate a differential expression profile; and
(c) comparing the differential expression profile with one or more reference differential expression profiles indicative of one or more stages of chronic/sclerosing allograft nephropathy,
thereby assessing the onset of rejection of the transplanted organ in the subject.

2. A method of classifying the stage of chronic/sclerosing allograft nephropathy in a subject suffering from chronic rejection of a transplanted kidney, comprising the steps of:
(a) determining the level of expression in a post-transplantation sample from a subject of a combination of a plurality of genes selected from the group consisting of the genes identified in Table 1;
(c) comparing the level of gene expression of said plurality of genes in the post-transplantation sample with the magnitude of gene expression of the same genes in a control sample to generate a differential expression profile; and
(d) comparing the differential expression profile with one or more reference differential expression profiles indicative of one or more stages of chronic/sclerosing allograft nephropathy,
thereby classifying the stage of chronic/sclerosing allograft nephropathy in the subject.

3. A method according to claim 2 wherein the plurality of genes selected have a expression profile that distinguishes stage III CAN from earlier stages.

4. A method according to any one of claims 1 to 3 wherein said plurality of genes comprises SLC1A3, CD163, RDH12 and FLJ32569.

5. Use of a plurality of nucleic acid probes, each of which probes hybridizes specifically to a different gene in Table 1 as a biomarker for chronic/sclerosing allograft nephropathy.

6. Use according to claim 5 wherein the plurality of nucleic acid probes are capable of detecting expression of the SLC1A3, CD163, RDH12 and FLJ32569 genes.
